# EUROPEAN PATENT APPLICATION

(11) **EP 4 458 272 A1**
(43) Date of publication of application: **06.11.2024**
(21) Application number: 23740311.8
(22) Date of filing: 12.01.2023
(51) Int. Cl.: A61B 8/00, A61B 17/34

(54) **PUNCTURE NEEDLE**

(30) Priority: 13.01.2022 JP 2022003960
(71) Applicant: Terumo Kabushiki Kaisha, Tokyo 151-0072 (JP)
(72) Inventor: FUJIKI Jo, Ashigarakami-gun, Kanagawa 259-0151 (JP); OKAMURA Ryo, Ashigarakami-gun, Kanagawa 259-0151 (JP); KAMBARA Kayo, Ashigarakami-gun, Kanagawa 259-0151 (JP)
(74) Representative: TBK
(86) International application number: PCT/JP2023/000658
(87) International publication number: WO 2023/136303

(57) **Abstract**

Provided is a puncture needle with improved echo visibility in a state where a probe of an ultrasound diagnostic device is brought into contact in a direction along an axial direction of the puncture needle from a proximal side of the puncture needle. The puncture needle includes: a main body having a rod shape; a needle tip formed at a distal portion of the main body; and a recess formed in a side portion of the main body, wherein the recess has a distal-side wall that has a wall surface facing the proximal side and facing a direction of being warped at 0° or more and 75° or less from a direction along an axis of the main body to a radially outer side of the main body, and the distal-side wall and the main body are separated from each other when viewed in a normal direction of the distal-side wall.

## Description

### Technical Field

The present disclosure relates to a puncture needle.

### Background Art

JP H3-228748 A (Patent Literature 1) discloses an ultrasound reflector including a needle or a pipe inserted into an object while confirming an image of a deep portion in an ultrasound guide, the needle or the pipe having an insertion portion whose surface is formed uneven. As the ultrasound reflector, one having a V-groove formed on a surface of an outer needle is described. The V-groove has a groove angle of around 90°, and as the V-groove, one formed such that each of two sides has 45° with respect to the surface of the outer needle and one having one side having an angle smaller than 45° and the other side having an angle larger than 45° are described.

JP 2019-208962 A (Patent Literature 2) describes a follicular growth inducing device. The follicular growth inducing device includes: an ovarian puncture needle that forms a puncture hole by puncturing an ovary in a traveling direction of ultrasound emitted from a probe in a transvaginal ultrasound device toward the ovary; an optical fiber that guides a laser beam emitted from a laser generator; a guide being tubular to receive a needle tube of the ovarian puncture needle; and a fastener that fastens the guide to the probe. The guide is fastened to the probe in a manner elongated in the traveling direction of the ultrasound toward the ovary.

### Citation List

### Patent Literature

Patent Literature 1: JP H3-228748 A
Patent Literature 2: JP 2019-208962 A

### Summary of Invention

### Technical Problem

As described in Patent Literature 1, there is a case where a structure that reflects ultrasound is formed on an outer surface of a puncture needle in consideration of echo visibility regarding puncture under an echo. However, there is room for improvement in the echo visibility in a case where puncture is performed in the traveling direction of the ultrasound using the puncture needle as described in Patent Literature 2, that is, in a case where a procedure is performed in a state where the probe of an ultrasound diagnostic device is brought into contact in a direction along an axial direction of the puncture needle from a proximal side of the puncture needle.

The present disclosure has been made in view of such reality, and an object thereof is to provide a puncture needle with improved echo visibility in a state where a probe of an ultrasound diagnostic device is brought into contact in a direction along an axial direction of the puncture needle from a proximal side of the puncture needle.

### Solution to Problem

In order to achieve the above object, a puncture needle according to the present disclosure includes: a main body having a rod shape; a needle tip formed at a distal portion of the main body; and a recess formed in a side portion of the main body, wherein the recess has a distal-side wall that has a wall surface facing a proximal side and facing a direction of being warped at 0° or more and 75° or less from a direction along an axis of the main body to a radially outer side of the main body, and the distal-side wall and the main body are separated from each other when viewed in a normal direction of the distal-side wall.

Further, in the puncture needle according to the present disclosure, the distal-side wall is directed in a direction of being warped to the radially outer side of the main body from an orientation directly facing the back on the proximal side.

Further, in the puncture needle according to the present disclosure, a virtual line connecting a bottom-side end of the distal-side wall and a proximal end of the recess in the direction along the axis intersects the distal-side wall at an obtuse angle.

Further, in the puncture needle according to the present disclosure, the recess has the distal-side wall having a planar shape and a proximal-side wall that has a planar shape and extends from the bottom-side end of the distal-side wall to the proximal end of the recess, and the proximal-side wall is formed along the virtual line.

Further, in the puncture needle according to the present disclosure, the recess is formed in the needle tip.

Further, in the puncture needle according to the present disclosure, the needle tip has a blade surface obtained by cutting a distal portion of the main body obliquely with respect to the direction along the axis, and the recess is disposed at the same position as the blade surface in the direction along the axis.

### Advantageous Effects of Invention

According to the present disclosure, it is possible to provide the puncture needle with the improved echo visibility in the state where the probe of the ultrasound diagnostic device is brought into contact in the direction along the axial direction of the puncture needle from the proximal side of the puncture needle.

### Brief Description of Drawings

Fig. 1 is a side view of a puncture needle according to the present embodiment.
Fig. 2 is an enlarged view of the vicinity of a recess in Fig. 1.
Fig. 3 is a view illustrating the vicinity of the recess as viewed in a direction of an arrow A in Fig. 2.
Fig. 4 is a view illustrating a mode in which the puncture needle is used under an echo.
Fig. 5 is a top view of the vicinity of the recess.
Fig. 6 is an enlarged view of the vicinity of a recess according to a first modification.
Fig. 7 is a side view of a puncture needle according to a second modification.
Fig. 8 is an enlarged view of the vicinity of a recess in Fig. 7.
Fig. 9 is a top view of the vicinity of the recess according to the second modification.
Fig. 10 is a side view of a puncture needle according to a third modification.
Fig. 11 is a side view of a puncture needle according to a fourth modification.

### Description of Embodiments

A puncture needle according to an embodiment of the present disclosure is described with reference to the drawings.

### (Description of Outline)

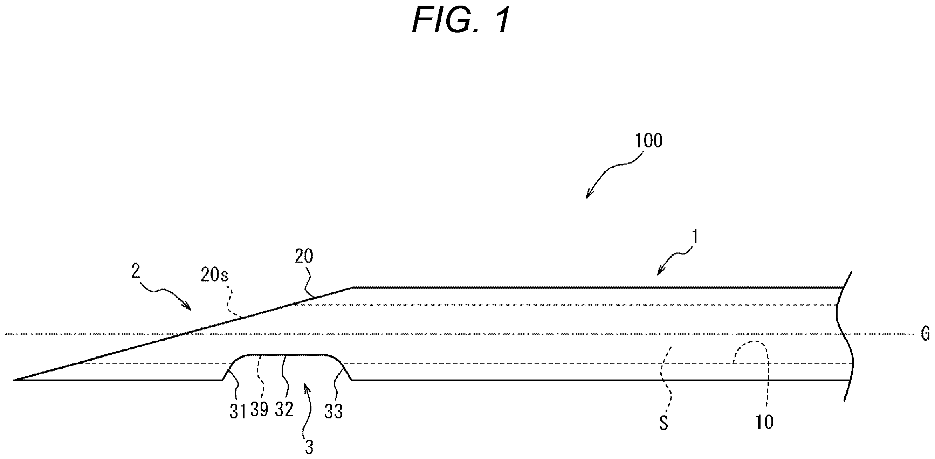

As illustrated in Fig. 1, a puncture needle 100 according to the present embodiment includes a main body 1 having a rod shape, a needle tip 2 formed at a distal portion of the main body 1, and a recess 3 formed in a side portion of the main body 1. Note that Fig. 1 is a side view of the puncture needle 100.

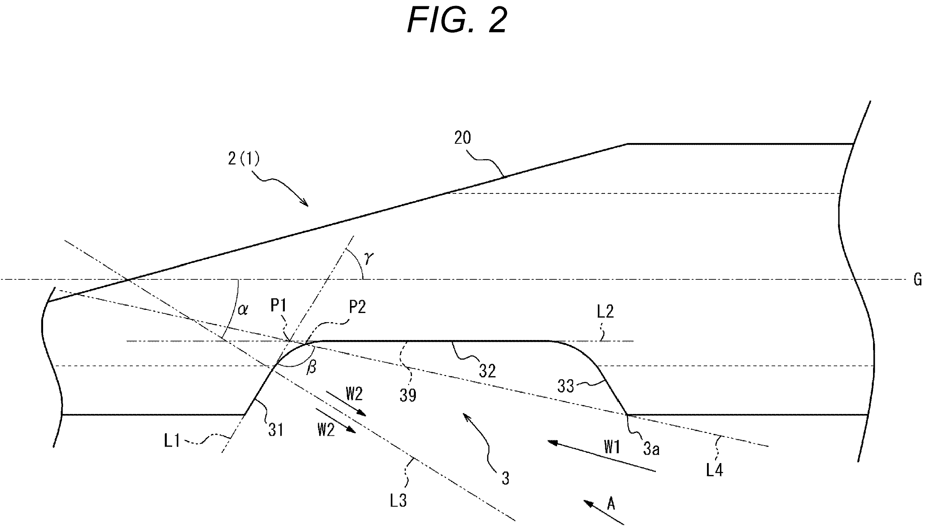

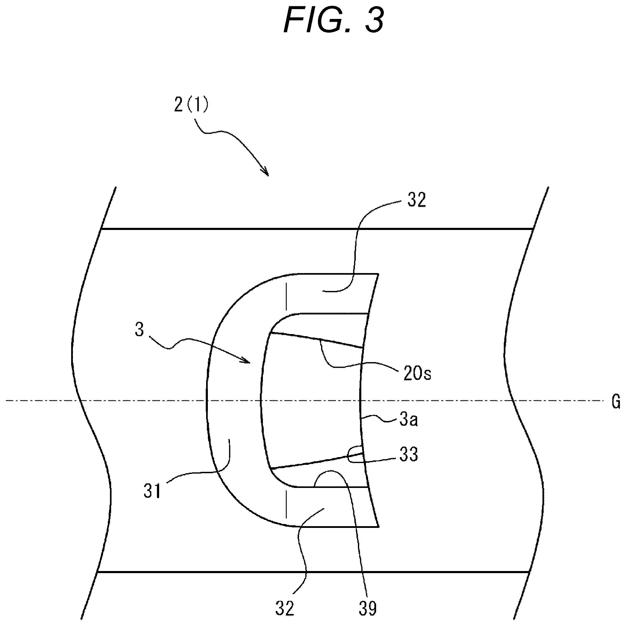

As illustrated in Fig. 2, the recess 3 has a distal-side wall 31 having a wall surface facing a proximal side and facing a direction of being warped at 0° or more and 75° or less from a direction along an axis G of the main body 1 to the radially outer side of the main body 1. When viewed in a normal direction of the distal-side wall 31, the distal-side wall 31 and the main body 1 (a boundary 3a to be described later) on the proximal side of the recess 3 are separated from each other (see Fig. 3). Note that Fig. 2 is a partially enlarged view illustrating the vicinity of the recess 3 in Fig. 1 in an enlarged manner. Fig. 3 is a view in which the recess 3 is viewed in the normal direction of the distal-side wall 31, that is, the recess 3 is viewed in a direction of an arrow A in Fig. 2.

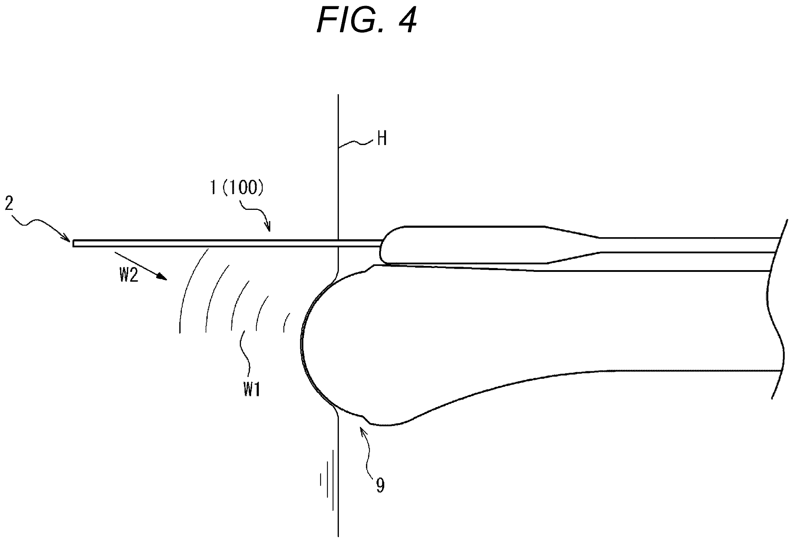

As illustrated in Fig. 4, the puncture needle 100 is suitable for a procedure in a state where a probe 9 of an ultrasound diagnostic device is brought into contact with a body surface H of a living body in the direction along the axis G of the main body 1 of the puncture needle 100 from the proximal side of the puncture needle 100. When the procedure is performed, the puncture needle 100 has improved echo visibility as will be described later.

### (Description of Each Part)

Hereinafter, each part of the puncture needle 100 will be described.

As illustrated in Fig. 1, the main body 1 has a cylindrical tubular shape as an example of the rod shape. A space defined by an inner wall 10 of the tubular inside of the main body 1 is a lumen S.

The main body 1 may be made of, for example, a metal such as stainless steel, titanium, a titanium alloy, or a cobalt-chromium alloy or a synthetic resin such as a fluororesin or a polyolefin resin. In the present embodiment, a case where the main body 1 is made of stainless steel is exemplified.

The needle tip 2 is formed at a distal end of the main body 1. The needle tip 2 has, for example, a shape having a blade surface 20 obtained by cutting the distal portion of the main body 1 obliquely with respect to the direction along the axis G. The needle tip 2 may have a shape other than the shape having the blade surface 20 obtained by obliquely cutting the distal portion of the main body 1.

The lumen S is a space continuously formed along the axis G inside the main body 1. In the present embodiment, the lumen S is formed as a cylindrical space whose axis overlaps the axis G and whose cross section orthogonal to the axis G has a circular shape. In the present embodiment, a case where the lumen S communicates with an external space via an opening 20s formed in the blade surface 20 is exemplified.

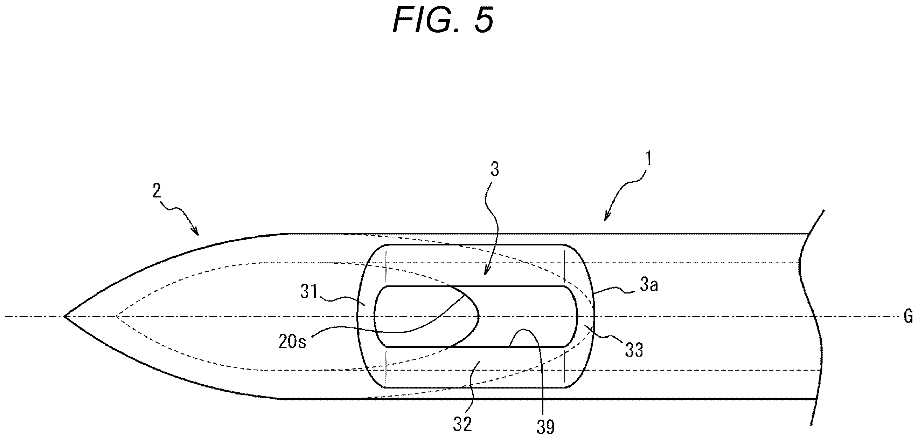

The recess 3 is formed in the side portion of the main body 1. The recess 3 is recessed in a direction intersecting the axis G. The recess 3 may be formed in a trapezoidal shape, a V-shape, or the like in which the radially outer side of the main body 1 is widened in the direction along the axis G in the side view of the puncture needle 100. Figs. 1 and 2 illustrate a case where the recess 3 is formed in the trapezoidal shape. In the present embodiment, as illustrated in Fig. 5, the recess 3 is formed in a range from an outer peripheral surface of the main body 1 to reach the lumen S, and an opening 39 communicating with the lumen S is formed in the recess 3. That is, the recess 3 penetrates to the lumen S. Note that Fig. 5 is a top view of the vicinity of the recess 3 in the puncture needle 100, and is a view of the puncture needle 100 in the state illustrated in Figs. 1 and 2 as viewed from the bottom to the top in Fig. 1.

As illustrated in Figs. 1, 2, and 5, the recess 3 is formed in the needle tip 2 in the present embodiment. That is, the recess 3 is disposed at the same position as the needle tip 2 in the direction along the axis G. When description is made in relation to the blade surface 20, the recess 3 is disposed at the same position as the blade surface 20 in the direction along the axis G. As a result, the visibility under an echo of the needle tip 2 is improved. The recess 3 is preferably formed in the main body 1 on the opposite side of the blade surface 20 with respect to the axis G.

As illustrated in Figs. 2, 5, and the like, the recess 3 includes a bottom wall 32 serving as a bottom surface, the distal-side wall 31 which is located on a distal side of the bottom wall 32 and extends from the outer peripheral surface of the main body 1 to the bottom wall 32, and a proximal-side wall 33 which is located on the proximal side of the bottom wall 32 and extends from the outer peripheral surface of the main body 1 to the bottom wall 32.

The bottom wall 32 has a planar bottom surface along the axis G. The opening 39 (see Fig. 3) communicating with the lumen S is formed in the bottom wall 32. The opening 39 may be used as a so-called back eye in the puncture needle 100.

The distal-side wall 31 is a planar wall that is the outer peripheral surface of the main body 1 and extends from a distal end of the recess 3 to the bottom wall 32. The planar portion of the distal-side wall 31 is slightly inclined to the radially outer side of the main body 1 from an orientation facing the proximal side in the direction along the axis G. That is, the distal-side wall 31 is directed in a direction of being warped to the radially outer side of the main body 1 from an orientation directly facing the back on the proximal side of the main body 1.

The proximal-side wall 33 is a planar wall which is the outer peripheral surface of the main body 1 and extends from a proximal end of the recess 3 to the bottom wall 32. The planar portion of the proximal-side wall 33 is slightly inclined to the radially outer side of the main body 1 from an orientation facing the distal side in the direction along the axis G. That is, the proximal-side wall 33 is directed in a direction of being warped to the radially outer side of the main body 1 from an orientation directly facing the front on the distal side of the main body 1.

In Fig. 2, a virtual line along the wall surface of the distal-side wall 31, the line intersecting the axis G, is illustrated as a virtual line L1 in a side view (a side view in a state illustrated in Fig. 2 in the present embodiment, referred to as a predetermined side view hereinafter) in a case where the puncture needle 100 is viewed from a predetermined circumferential position with respect to the axis G (the main body 1). Note that the predetermined side view in the present embodiment is a viewpoint of viewing the recess 3 from a side surface.

In Fig. 2, in the predetermined side view, a virtual line along a wall surface of the bottom wall 32, the line being located on the same plane as the axis G and the virtual line L1, is illustrated as a virtual line L2.

The planar portion of the wall surface of the distal-side wall 31 overlapping the virtual line L1 is directed in the direction of being warped to the radially outer side of the main body 1 from the orientation facing the proximal side, that is, the orientation directly facing the back. As a result, the distal-side wall 31 can reflect ultrasound W1 from the probe 9 to the proximal side as a reflected wave W2. In Fig. 2, a virtual line along a normal line of the virtual line L1, that is, the distal-side wall 31, is illustrated as a virtual line L3.

In Fig. 2, an angle at which the virtual line L1 intersects the axis G is indicated by an intersection angle γ, and the intersection angle γ is 15° or more and 90° or less. That is, the distal-side wall 31 intersects the axis G at the angle of 15° or more and 90° or less.

In Fig. 2, an angle at which the virtual line L3 along the normal line of the virtual line L1 (the distal-side wall 31) intersects the axis G is indicated by an intersection angle α. That is, the virtual line L3 along the normal line of the virtual line L1 intersects the axis G at 0° or more and 75° or less. In other words, the distal-side wall 31 overlapping the virtual line L1 is directed toward the orientation directly facing the back on the proximal side and in the direction of being warped at 0° or more and 75° or less from the direction along the axis G to the radially outer side of the main body 1.

A virtual line L4 that connects the boundary 3a between the proximal-side wall 33 and an outer surface of the main body 1, which is a proximal end of the recess 3 located on a plane overlapping the axis G, and a bottom-side end of the distal-side wall 31 is formed to intersect the virtual line L1, that is, the distal-side wall 31 at an obtuse angle (more than 90° and less than 180°). Note that the bottom-side end of the distal-side wall 31 is a boundary position between the distal-side wall 31 and the bottom wall 32.

A case where a boundary portion between the distal-side wall 31 and the bottom wall 32 is formed in a curved surface shape is illustrated in the present embodiment. In such a case, as illustrated in Fig. 2, a position P2 where a virtual line (the same as the virtual line L4 in the present embodiment) connecting the boundary 3a and an intersection P1 between the virtual line L2 and the virtual line L1 intersects the boundary portion between the distal-side wall 31 and the bottom wall 32 is defined as the boundary position between the distal-side wall 31 and the bottom wall 32.

In Fig. 2, an angle at which the virtual line L4 intersects the virtual line L1 is indicated by an intersection angle β. That is, the intersection angle β is the obtuse angle (more than 90° and less than 180°). As a result, it is possible to avoid the bottom wall 32 and the proximal-side wall 33 from hindering incidence of the ultrasound W1 (see Fig. 4) on the distal-side wall 31. In addition, it is possible to avoid the bottom wall 32 and the proximal-side wall 33 from shielding the reflected wave W2 (see Fig. 4). As a result, it is possible to improve the echo visibility of the puncture needle 100 in the state where the probe 9 (see Fig. 4) of the ultrasound diagnostic device is brought into contact in the direction along the axis G of the puncture needle 100 from the proximal side of the puncture needle 100. Note that the intersection angle β is preferably more than 120° and less than 170°. When the intersection angle β falls within such a range, the echo visibility of the puncture needle 100 can be improved even if the probe 9 slightly deviates from the direction along the axis G of the puncture needle 100 from the proximal side of the puncture needle 100 under an echo in a generally assumed procedure.

In addition, since the distal-side wall 31 is formed in the planar shape, the ultrasound W1 incident on the distal-side wall 31 is reflected without being diffused. Therefore, the reflected wave W2 is reflected with large energy toward the proximal side. As a result, it is possible to further improve the echo visibility of the puncture needle 100 in the state where the probe 9 (see Fig. 4) of the ultrasound diagnostic device is brought into contact in the direction along the axis G of the puncture needle 100 from the proximal side of the puncture needle 100. Note that the distal-side wall 31 may be formed in a curved surface shape. The distal-side wall 31 formed in the curved surface shape can reflect the ultrasound W1 incident at a plurality of angles (various angles).

### (Description of Modifications)

In the above-described embodiment, the description has been made by exemplifying the case where the recess 3 has the trapezoidal shape in which the radially outer side of the main body 1 is widened in the direction along the axis G in the side view of the main body 1 and the recess 3 penetrates to the lumen S. However, the recess 3 is not limited to the trapezoidal shape. In addition, the recess 3 does not necessarily penetrate to the lumen S.

### (First Modification)

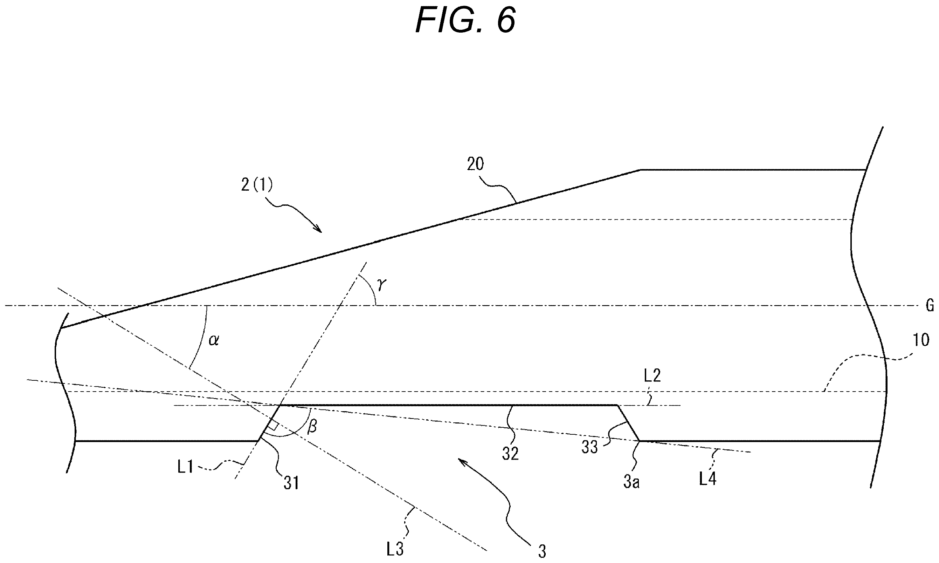

Fig. 6 is an enlarged view of the vicinity of the recess 3 in a case where the recess 3 does not penetrate to the lumen S. In this manner, the recess 3 does not necessarily penetrate to the lumen S.

### (Second Modification)

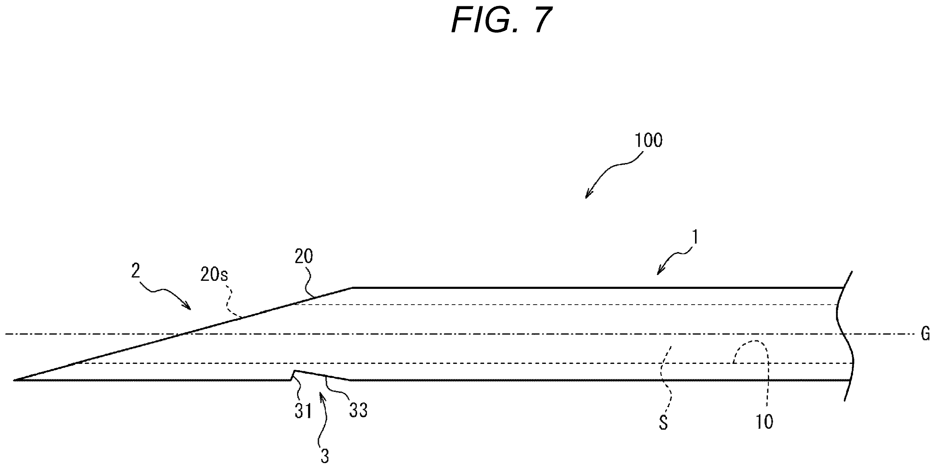

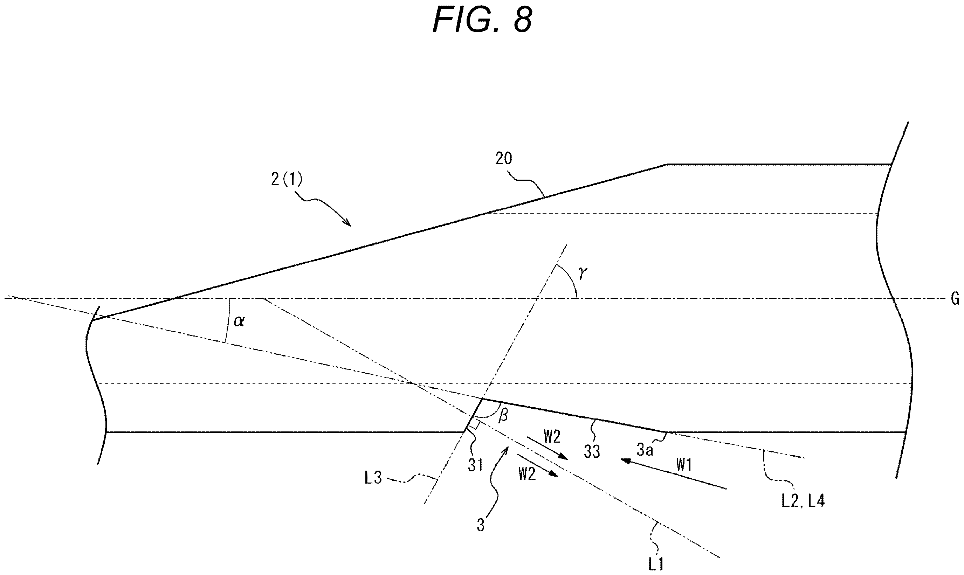

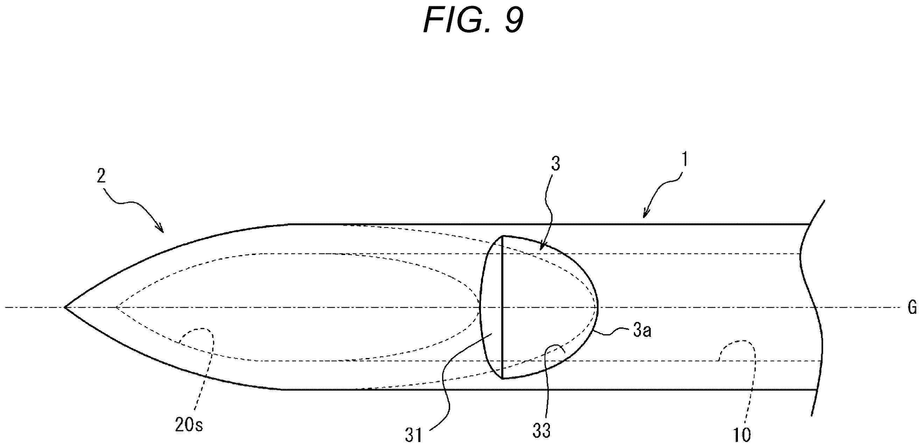

Figs. 7 and 8 illustrate a case where the recess 3 has a V-shape in a side view of the main body 1. The recess 3 is formed of the distal-side wall 31 having a planar shape and the proximal-side wall 33 having a planar shape. Fig. 9 illustrates a top view of the vicinity of the recess 3. That is, Fig. 9 is a view of the puncture needle 100 in a state illustrated in Figs. 7 and 8 as viewed from the bottom to the top in Fig. 1.

When the recess 3 is formed in the V-shape in the side view of the main body 1 In this manner, the above-described bottom wall 32 (see Fig. 2 and the like) is flush with the proximal-side wall 33. In the following description, when the bottom wall 32 is flush with the proximal-side wall 33, the description is made considering that the proximal-side wall 33 includes the above-described bottom wall 32. For example, the above-described virtual line L2 (see Fig. 2) is treated as a line along the proximal-side wall 33.

In the present modification, the bottom-side end of the distal-side wall 31 is connected to a bottom-side end of the proximal-side wall 33. In other words, the proximal-side wall 33 is formed as a planar wall extending from the bottom-side end of the distal-side wall 31 to a proximal end of the recess 3. In addition, in this case, the proximal-side wall 33 preferably overlaps the virtual line L4 connecting the boundary 3a between the proximal-side wall 33 and an outer surface of the main body 1 and the bottom-side end of the distal-side wall 31. As a result, it is possible to avoid the proximal-side wall 33 from hindering incidence of the ultrasound W1 (see Fig. 4) on the distal-side wall 31. In addition, it is possible to avoid the proximal-side wall 33 from shielding the reflected wave W2 (see Fig. 4). As a result, it is possible to improve the echo visibility of the puncture needle 100 in the state where the probe 9 (see Fig. 4) of the ultrasound diagnostic device is brought into contact in the direction along the axis G of the puncture needle 100 from the proximal side of the puncture needle 100.

### (Third Modification)

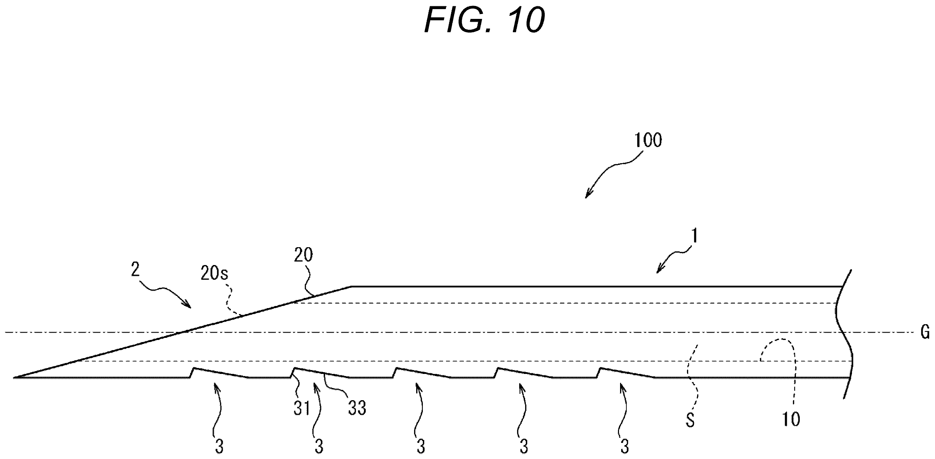

As illustrated in Fig. 10, a plurality of the recesses 3 may be formed in the main body 1. Therefore, it is possible to further improve the echo visibility of the puncture needle 100 in the state where the probe 9 (see Fig. 4) of the ultrasound diagnostic device of the ultrasound diagnostic device is brought into contact in the direction along the axis G of the puncture needle 100 from the proximal side of the puncture needle 100.

In this case, at least one of the plurality of recesses 3 may be disposed at the same position as the needle tip 2 (in Fig. 10, the blade surface 20) in the direction along the axis G. As a result, the visibility under an echo of the needle tip 2 is improved.

### (Fourth Modification)

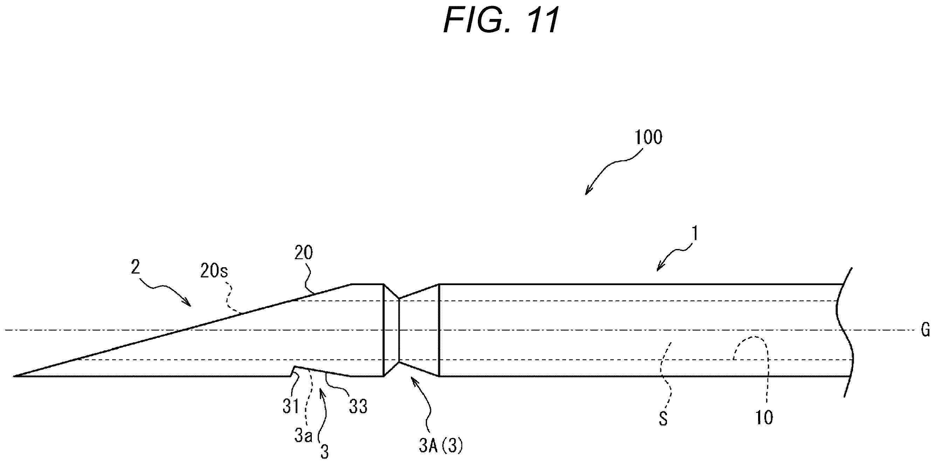

As illustrated in Fig. 11, for example, a recess 3A formed in an annular shape along a circumferential direction of the main body 1 may be formed as the recess 3. One or more recesses 3A may be formed in the main body 1. Therefore, the echo visibility of the puncture needle 100 can be improved regardless of any orientation of the puncture needle 100 in the circumferential direction in the state where the probe 9 (see Fig. 4) of the ultrasound diagnostic device is brought into contact in the direction along the axis G of the puncture needle 100 from the proximal side of the puncture needle 100.

Note that the example illustrated in Fig. 11 illustrates a case where the puncture needle 100 includes the recess 3 disposed on the distal side in the direction along the axis G and the recess 3A disposed on the proximal side. The recess 3 disposed on the distal side is disposed at the same position as the needle tip 2. The recess 3A disposed on the proximal side is disposed on the proximal side of the needle tip 2. In the above-described predetermined side view, which is the viewpoint of viewing the recess 3 on the distal side from the side surface, the recess 3 on the distal side and the recess 3A, which is the recess 3 on the proximal side, may have similar shapes. In the example illustrated in Fig. 11, the recess 3 on the distal side and the recess 3A, which is the recess 3 on the proximal side, have the same shape in the predetermined side view.

Note that the recess 3 may be formed in a spiral shape along the circumferential direction of the main body 1 although not illustrated. Also in this case, the echo visibility of the puncture needle 100 can be improved regardless of any orientation of the puncture needle 100 in the circumferential direction in the state where the probe 9 (see Fig. 4) of the ultrasound diagnostic device is brought into contact in the direction along the axis G of the puncture needle 100 from the proximal side of the puncture needle 100.

As described above, it is possible to provide the puncture needle with the improved echo visibility in the state where the probe of the ultrasound diagnostic device is brought into contact in the direction along an axial direction of the puncture needle from the proximal side of the puncture needle.

### [Other Embodiments]

(1) Although the case where the main body 1 has the tubular shape including the lumen S has been described as an example in the above-described embodiment, the main body 1 may have a solid rod shape.
(2) The case where the needle tip 2 has, for example, the shape having the blade surface 20 obtained by cutting the distal portion of the main body 1 obliquely with respect to the direction along the axis G has been described in the above-described embodiment. However, the needle tip 2 can also have, for example, a pyramid shape. An example of the pyramid shape is a conical shape or a polygonal pyramid shape including a plurality of planar portions on side faces. Examples of the polygonal pyramid shape include a triangular pyramid shape, a quadrangular pyramid shape, and a pentagonal or more pyramid shape.

Note that the configurations disclosed in the above-described embodiments (including the other embodiments, the same applies hereinafter) can be applied in combination with configurations disclosed in other embodiments as long as there is no contradiction, and the embodiments disclosed in the present specification are examples, and the embodiments of the present disclosure are not limited thereto, and can be appropriately modified within a scope not departing from the object of the present disclosure.

### Industrial Applicability

The present disclosure is applicable to a puncture needle.

### Reference Signs List

- 1: Main body
- 10: Inner wall
- 100: Puncture needle
- 2: Needle tip
- 20: Blade surface
- 20s: Opening
- 3: Recess
- 31: Distal-side wall
- 32: Bottom wall
- 33: Proximal-side wall
- 39: Opening
- 3A: Recess
- 3a: Boundary
- 9: Probe
- G: Axis
- H: Body surface
- L1: Virtual line
- L2: Virtual line
- L3: Virtual line
- L4: Virtual line
- P1: Intersection
- P2: Position
- S: Lumen
- W1: Ultrasound
- W2: Reflected wave
- α: Intersection angle
- β: Intersection angle
- γ: Intersection angle

## Claims

1. A puncture needle comprising:
a main body having a rod shape;
a needle tip formed at a distal portion of the main body; and
a recess formed in a side portion of the main body, wherein
the recess has a distal-side wall that has a wall surface facing a proximal side and facing a direction of being warped at 0° or more and 75° or less from a direction along an axis of the main body to a radially outer side of the main body, and
the distal-side wall and the main body are separated from each other when the distal-side wall and the main body are viewed in a normal direction of the distal-side wall.

2. The puncture needle according to claim 1, wherein the distal-side wall is directed in a direction of being warped to the radially outer side of the main body from an orientation directly facing a back on the proximal side.

3. The puncture needle according to claim 1 or 2, wherein a virtual line connecting a bottom-side end of the distal-side wall and a proximal end of the recess in the direction along the axis intersects the distal-side wall at an obtuse angle.

4. The puncture needle according to claim 3, wherein
the recess has the distal-side wall having a planar shape and a proximal-side wall that has a planar shape and extends from the bottom-side end of the distal-side wall to the proximal end of the recess, and
the proximal-side wall is formed along the virtual line.

5. The puncture needle according to any one of claims 1 to 3, wherein the recess is formed in the needle tip.

6. The puncture needle according to claim 5, wherein
the needle tip has a blade surface obtained by cutting a distal portion of the main body obliquely with respect to the direction along the axis, and
the recess is disposed at a position identical to a position of the blade surface in the direction along the axis.
